# EUROPÄISCHE PATENTANMELDUNG

(11) **EP 1 099 415 A1**
(43) Veröffentlichungstag der Anmeldung: **16.05.2001**
(21) Anmeldenummer: 99122429.6
(22) Anmeldetag: 10.11.1999
(51) Int. Cl.: A61B 17/66

(54) **Knochendistraktor mit einer Stelleinrichtung**

(71) Anmelder: Longerich, Ulrich, Dr. Dr., 82031 Grünwald (DE)
(72) Erfinder: Longerich, Ulrich, Dr. Dr., 82031 Grünwald (DE); Janzen, Wolfgang, Dipl.-Ing., 67346 Speyer (DE); Thurau, Matthias, Dr. Dr., 83109 Grosskarolinenfeld (DE)
(74) Vertreter: Castell, Klaus, Dr.

(57) **Zusammenfassung**

Der Knochendistraktor weist eine Stelleinrichtung auf, die mindestens zwei Knochenhalterungen hat. Diese Stelleinrichtung wird über eine Antriebseinheit mindestens einer Antriebsmaschine bewegt. Hierbei wird eine Knochenhalterung relativ zu der anderen Knochenhalterung entlang einer Bewegungsbahn bewegt. Diese Bewegungsbahn umfaßt mindestens eine geradlinige und eine weitere Bewegungskomponente.

Vorteilhaft ist es, wenn die Knochenhalterungen Hebel aufweisen, die an mindestens zwei Punkten angelängt sind. Die Verwendung einzeln steuerbarer Antriebsmaschinen ermöglicht eine individuelle vorbestimmbare Bewegung, die auch über einen Sender und einen Empfänger gesteuert werden kann.

## Beschreibung

Die Erfindung betrifft einen Knochendistraktor mit einer Stelleirichtung, die mindestens zwei Knochenhalterungen aufweist, und eine Antriebseinheit mit mindestens einer Antriebsmaschine.

Ein derartiger Knochendistraktor ist aus dem US Patent Nummer 5,334,202 bekannt. Hierbei wirkt ein Motor auf eine Führungsmechanik und die Führungsmechanik weist fest mit zwei Knochenstücken verbundene Knochenhalterungen auf. Dadurch ist es möglich, mittels der Antriebskraft des Motors die Knochenstücke gezielt auseinander zu drücken.

Ein weiterer gattungsgemäßer Knochendistraktor ist aus der EP 0809 473 B1 bekannt. Dieser Knochendistraktor weist zwei Knochenhalterungen auf, die auf einer geraden Linie angeordnet sind. Eine Knochenhalterung bildet eine Hülse und die andere Knochenhalterung einen darin geführten Stab. In der Hülse ist ein Motor angeordnet, mit dem der Stab relativ zur Hülse bewegt werden kann. Auch mit dieser Vorrichtung ist es möglich zwei Knochen auf einer geraden Linie auseinander zu bewegen.

Diese bekannten Knochendistraktoren sind vorallem für Röhrenknochen geeignet und führen zur einer monodirektionalen Knochenverlängerung. Insbesondere für die osteogenetische Distraktion von Knochen im Mund-Kiefer-Gesichtsbereich sind diese Distraktoren jedoch nicht geeignet, da sie im Hinbick auf ihre Form, ihre Größe und ihre Funktion den Anforderungen im Mund-Kieferbereich nicht gewachsen sind. Besonders komplizierte Knochenbewegungen werden beispielsweise bei "fliehendem Kinn" oder bei Gesichtsoperationen nach Tumorbehandlungen notwendig. Für derartig komplizierte osteogenetische Distraktionen werden bisher manuell verstellbare Distraktoren verwendet. Zur Einstellung einer individuellen Okklusion, das heißt der Verschlüsselung der Zahnbögen des Oberkiefers und des Unterkiefers zueinander, sind die bekannten Distraktoren viel zu grob in der Einstellung und extrem schwer einstellbar.

Die bekannten Distraktoren sind meist auch nicht kontinuierlich verstellbar. Die Verstellung des Distraktors in diskreten Schritten fügt dem Patienten bei der Verstellung Dehnungsschmerzen zu. Um diese Dehnungsschmerzen gering zu halten, muß der Patient in regelmäßigen, möglichst kurzen Abständen den Chirurgen besuchen, um den Distraktor in möglichst kleinen Schritten nachzustellen. Auch dies ist jedoch unangenehm, zeit- und kostenaufwendig.

Infolge von Einsparungen auf dem Medizinsektor werden auch bei den bekannten manuell verstellbaren Distraktoren die Nachstellintervalle möglichst groß bemessen. Da das Knochenwachstum nicht immer in der gleichen Geschwindigkeit erfolgt, entstehen somit Ungenauigkeiten im Bereich der Knochenneubildung und besonders hohe Dehnungsschmerzen beim Nachstellen des Distraktors.

Der Erfindung liegt daher die Aufgabe zugrunde, einen gattungsgemäßen Knochendistraktor so weiterzubilden, daß er auch im Mund-Kiefer-Gesichtsbereich einsetztbar ist.

Diese Aufgabe wird bei einem Knochendistraktor mit einer Stelleinrichtung, die mindestens zwei Knochenhalterungen aufweist, und einer Antriebseinheit mit mindestens einer Antriebsmaschine gelöst, indem eine Knochenhalterung relativ zur anderen Knochenhalterung entlang einer Bewegungsbahn bewegbar ist, die zumindest eine geradlinige und eine weitere Bewegungskomponente umfaßt.

Der Erfindung liegt die Erkenntnis zugrunde, daß ein Antrieb einer chirurgischen Einrichtung nicht unbedingt in einer geradlinigen Bewegungsrichtung erfolgen muß, sondern auch eine Bewegung entlang einer Bewegungsbahn bewirken kann. Diese für Knochendistraktoren vorgeschlagene Bewegungsbahn umfaßt mindestens eine geradlinige und eine weitere Bewegungskomponente. Die geradlinige Bewegungskomponente führt dazu, daß die Knochen voneinander weg bewegt werden und die weitere Bewegungskomponente erlaubt eine zielgerichtete Bewegung in einer beliebigen Richtung im Raum.

Hierbei kann die weitere Bewegungskomponente sowohl eine laterale als auch eine rotatorische Bewegungskomponente sein. Beide Bewegungskomponenten überlagern sich zur einer Bewegung auf einer Bewegungsbahn, die eine freie Modellierung von Knochen erlaubt. Der Chirurg ist somit nicht an eine durch mechanische Einrichtungen vorgegebene Bewegungsrichtung gebunden, sondern kann die Knochenbewegung auf einer von ihm vorgegebenen Bewegungsbahn führen ohne sich durch mechanische Prämissen einengen zu lassen.

Die durch die überlagerten Bewegungskomponenten erzielte Knochenbewegung ermöglicht dem Chirurgen die Knochenneubildung genaustens nach seinen Vorgaben einzuleiten und die Antriebsmaschine erlaubt eine kontinuierliche oder quasikontinuierliche Bewegung der Knochen. Gerade die kontinuierliche oder die quasikontinuierliche Bewegung der Knochen führt dazu, daß Dehnungsschmerzen nicht mehr entstehen und bei genauer Vorbestimmung der Bewegungsbahn können die Zeitabstände innerhalb derer Nachuntersuchungen durchzuführen sind, deutlich verlängert werden.

Bei einer genauen - vorzugsweise mit einem Computer durchgeführten-Vorberechnung der Bewegungsbahn führt der erfindungsgemäße Knochendistraktor zu einer Erhöhung der Präzision des medizinischen Eingrifffs, während gleichzeitig der Arbeitseinsatz des medizinischen Personals vermindert werden kann.

Zum Beispiel mit der multidirektionalen osteogenetischen Kallusdistraktion können die Kiefer so gesteuert werden, daß nach der Distraktion der Oberkiefer und der Unterkiefer so positioniert sind, daß eine individuell optimale Okklusion erreicht wird oder die Basis für eine kieferorthopädische Weiterbehandlung zur Einstellung einer individuell optimalen Okklusion gegeben ist. Dies ist mit den bekannten Methoden der Kallusdistraktion nicht möglich. Die unidirektionale Kallusdistraktion kann die Okklusion nicht im notwendigen Maße berücksichtigen, weil zur optimalen Okklusionseinstellung bei der Kallusdistraktion immer eine rotatorische Bewegungskomponente mit der unilateralen Bewegung kombinieret sein muß.

Durch die Addition einer vertikalen Rotationskomponente während der Distraktion zusätzlich zu der sagittalen, unidirektionalen Komponente kann die individuell optimale Okklusion ohne Zweitoperation eingestellt werden. Diese kombinierte, multidirektionale Distraktion wird mittels eines Computers vorberechnet und die Verlängerung des Kiefers unter dem Aspekt einer individuell optimalen Einstellung der Okklusion durchgeführt, so daß auf eine weitere Okklusion verzichtet werden kann. Die multidirektionale osteogenetische Kallusdistraktion dient nicht nur der Knochengewinnung als Vorbereitung der kieferorthopädischen Operation, sondern der Knochenverlängerung mit Einstellung der optimalen Okklusion, was mit einer erheblichen Entlastung für den Patienten verbunden ist.

Eine einfache Ausführungsvariante des erfindungsgemäßen Knochendistraktors wird dadurch erzielt, daß zumindest eine Bewegungskomponente der Bewegungsbahn durch eine Kurvenbahn bewirkt wird. Die Kurvenbahn bildet eine mechanisch festgelegte Führung für die angetriebene Knochenhalterung oder die angetriebenen Knochenhalterungen. Somit kann durch die Festlegung der Kurvenbahn die während der Distraktion der Knochen vollzogene Bewegungsbahn definiert vorbestimmt werden. Eine derartige Kurvenbahn kann ein vorgefertigtes Führungsstück sein, das während der Bewegung der Knochenhalterung auf die Knochenhalterung einwirkt, um die Bewegungsrichtung zu beeinflussen. Die Kurvenbahn kann jedoch auch mechanisch verstellbar sein, so daß während der Knochendistraktion die Kurvenbahn noch variierbar ist, um beispielsweise Feineinstellungen vorzunehmen.

Um Anlagerungen oder Verschmutzungen im Bereich der Kurvenbahn zu vermeiden, wird vorgeschlagen die Kurvenbahn abzudecken. Dies kann beispielsweise durch ein elastisches Material geschehen, das einerseits die Kurvenbahn abdeckt und andererseits eine Bewegung der Knochenhalterung relativ zur Kurvenbahn erlaubt.

Vorteilhaft ist es, wenn die Knochenhalterungen Hebel aufweisen, die an mindestens zwei Punkten angelenkt sind. Sofern einer dieser Anlenkpunkte stationär ist und der andere Anlenkpunkt bewegbar ist, entsteht eine Bewegung auf einer Kreisbahn. Es können jedoch auch beide Anlenkpukte antreibbar sein oder ein Anlenkpunkt wird in einer Kurvenbahn geführt. Diese Möglichkeiten bestehen bei der einen Knochenhalterung und verständlicher Weise kann auch die andere Knochenhalterung entsprechend angelenkt werden.

Die Verwendung eines Hebels mit mindestens zwei Anlenkpunkten erlaubt einen einfachen mechanischen Aufbau, der eine komplizierte Bewegung der beiden Knochenhalterungen relativ zu einander ermöglicht. Während zwei Anlenkpunkte eine beliebige Variation der Bewegung innerhalb einer Ebene ermöglichen, bewirkt ein dritter Anlenkpunkt eine räumliche Bewegung.

Beispielsweise die genaue Berechnung einer Kurvenbahn ermöglicht es, mittels einer Antriebsmaschine die Knochenhalterungen relativ zueinander auf einer beliebigen Bewegungsbahn zu führen. Vorteilhaft ist es jedoch, wenn die Knochenhalterungen Hebel aufweisen, die mit mindestens zwei Antriebsmaschinen in Wirkverbindung stehen. Die Verwendung mehrerer Antriebsmaschinen ermöglicht es, die Verwendung von Kurvenbahnen einzuschränken und erschließt eine freiere Steuerung der Bewegungsrichtung sowie das nachträgliche Einwirken auf die Bewegungsrichtung durch eine Steuerung der Leistung der Antriebsmaschinen.

Ein kompakter Aufbau des Knochendistraktors entsteht dadurch, daß die Antriebseinheit mindestens zwei parallel oder koaxial angeordnete Gestänge aufweist. Die Gestänge können beispielsweise als Spindeln ausgebildet sein, um die von der Antriebsmaschine aufgebrachte Kraft zu untersetzen. Der parallele oder koaxiale Aufbau ermöglicht es, die Stelleinrichtung als kompakte Einheit auszubilden, die den Patienten möglichst wenig behindert. Durch eine geeignete Auswahl der Antriebseinheit, kann sogar eine Miniatunisierung des Knochendistraktors erreicht werden, die es ermöglicht, den Knochendistraktor als Implantant im oder in der Nähe des Knochens anzuordnen.

Da mechanische Führungen störanfällig sein können, wird vorgeschlagen, daß die Antriebseinheit einzeln steuerbare Antriebsmaschinen aufweist. Dies ermöglicht eine gesteuerte Bewegung auf einer beliebig definierbaren Bewegungsbahn. Der Bewegungsablauf kann hierbei vorprogrammiert sein oder auch während der Knochendistraktion auf einfache Art und Weise noch variiert werden. Dies ist gerade bei einer ungleichmäßigen Knochenbildung infolge von unterschiedlicher Durchblutung oder anderen Faktoren von besonderer Bedeutung.

Um eine kontinuierliche oder quasikontinuierliche Bewegung zu ermöglichen, kommen verschiedenartigste Antriebseinheiten infrage. Durch Aufbringen von Wärme, können Körper wie beispielsweise ein Piezoelememt ausgedehnt werden. Eine derartige Ausdehnung führt zu einer sehr hohen Kraft bei geringer Materiallängung. Sofern eine stärkere Materiallängung bewirkt werden soll als durch die Ausdehnung des Körpers erzielbar ist, kann der Körper auch mehrmals nacheinander ausgedehnt werden, wobei zwischen den Ausdehnungsphasen Abkühlungsphasen vorgesehen sind und beispielsweise durch eine Rasteinrichtung während jeder Ausdehnungsphase ein Stellglied um ein Intervall weitergeschoben wird. Die Erwärmung kann hierbei durch Wärmeeinwirkung von außen oder beispielsweise durch eine elektrische Heizspirale erzielt werden.

Eine andere Ausführungsform einer Antriebsmaschine sieht ein Uhrwerk vor. Ein Uhrwerk hat den Vorteil des kontinuierlichen Laufes, wobei es für den beschriebenen Anwendungsfall so ausgebildtet sein muß, daß größere Kräfte übertragen werden können. Uhrwerke haben darüberhinaus den Vorteil der extrem hohen Präzision und sie sind relativ klein herstellbar.

Um eine möglichst einfache Steuerung der Antriebseinheit zu ermöglichen wird in einer Version der Erfindung vorgeschlagen, daß die Antriebseinheit mindestens einen Elektro- oder Liniarmotor aufweist. Diese Motoren können gegebenenfalls mit einem Getriebe versehen sein, um die notwendigen kleinen Bewegungsstrecken bei hohen Gegenkräften zu bewirken. Außerdem sind derartige Motoren in derart miniaturisierter Form bekannt, daß sie sich hervorragend für implantierte Knochendistraktoren eignen.

Die Eintriebseinheit kann durch aus dem Körper herausgeführte Kabel kontinuierlich mit Energie versorgt werden. Vorteihaft ist es jedoch, wenn die Energieversorgung für die Antriebseinheit in der Nähe der Antriebseinheit angeordnet ist. Dies erlaubt die Ausbildung eines kompakten Knochendistraktors, der ohne Kabelanbindung autark arbeiten kann. Dies widerum erleichtert die Implantation des Distraktors und es erleichtert dem Patienten den Umgang mit einer derartigen Einrichtung. Als Energieversorgung können miniatursierte Batterien verwendet werden. Eine Ausführungsform sieht vor, daß ein Empfänger elektromagnetische Wellen aufnimmt und diese Energie in elektrischen Strom umwandelt, der in einer Batterie gespeichert wird. Dies erlaubt es, wiederaufladbare Batterien zu verwenden, die ohne Kabelverbindung mit elektrischem Strom aufgeladen werden können. Außerdem kann die Antriebseinheit direkt über die Stromversorgung gesteuert werden.

Hierzu wird vorgeschlagen, daß die Antriebseinheit einen Empfänger aufweist. Dieser Empfänger kann jedoch auch Steuersignale empfangen, die direkt eine Bewegung der Antriebsmaschine oder der Antriebsmaschinen bewirken. Die Steuersignale können jedoch auch der Programmierung einer im Knochendistraktor angeordneten Steuerung dienen, die widerum die Antriebsmaschine steuert. Dadurch wird ermöglicht, bei regelmäßigen ärztlichen Untersuchungen, die Programmierung der Steuereinrichtung des Knochendistraktors zu überprüfen und gegebenenfalls kleinere Änderungen einzuprogrammieren, um auf ein nicht genau vorprogrammierbares Knochenwachstum individuell einzugehen.

Letzlich wird vorgeschlagen, daß die Stelleinrichtung eine vorzugsweise mit einem Sender versehene Parametermeßeinrichtung aufweist. Dies ermöglicht es, beispielsweise Parameter zur Position der Knochenhalterungen bzw. der Knochen selbst zu ermitteln. Diese Meßwerte können entweder direkt zur Steuerung oder Regelung der Stelleinrichtung verwendet werden oder sie können mittels des Senders an eine Auswerteeinheit drahtlos übertragen werden, so daß beispielsweise mittels eines Computers die erreichte Position graphisch dargestellt werden kann und die folgenden Schritte berechnet werden können. Eine kontinuierliche oder quasikontinuierliche Messung der Positionsparameter der Stelleinrichtung erlaubt es, die Verstellung in Abhängigkeit von der Zeit nachzuvollziehen und sogar als Bewegtbild am Computer zu zeigen. Aus diesen Werten kann die zurückliegende Entwicklung nachverfolgt werden, um darauf basierend die zukünftige Entwicklung festzulegen. Sofern die Bewegungsrichtungen der Knochendistraktion anfänglich genau vorberechnet worden ist, kann anhand der gemessenen Werte die Entwicklung überprüft werden und es können gegebenenfalls Korrekturen bei der Verstellung des Knochendistraktors vorgesehen werden. Hierzu ist es von besonderem Vorteil, wenn die Antriebseinheit - wie oben erwähnteinen Empfänger aufweist. Hierdurch ist es sogar möglich einen Regelkreis aufzubauen, der es erlaubt, auf der Grundlage eines Vergleichs zwischen einem Sollwert und gemessenen Parametern die Antriebseinheit zu regeln.

Die Parametermeßeinrichtung kann auch die auf die Knochenhalterungen wirkende Kraft messen. Dies erfolgt beispielsweise mit einer Druckmeßeinrichtung, die zwischen den Knochenhalterungen angebracht ist. Dies ermöglicht es, in Abhängigkeit von der notwendigen Kraft die Antriebseinheit zu steuern oder zu regeln, um möglicherweise bei geringem Gegendruck schneller und bei hohem Gegendruck langsamer die Knochendistraktion vorzunehmen.

Die Parametermeßeinrichtung kann auch dazu dienen, den Sauerstoffpartialdruck im Blut, im Bereich der Knochenhalterungen, zu messen. Ein verringerter Sauerstoffpartialdruck weist hierbei auf eine zu geringe Durchblutung hin, die eine Folge von zu starkem Druck auf die Knochenhalterungen sein kann. Dies ermöglicht es den Sauerstoffpartialdruck im Blut bei der Regelung der Stelleinrichtung zu berücksichtigen.

Darüberhinaus können beliebige weitere Parameter, wie beispielsweise die Spannung der Stromversorgungseinrichtung gemessen und vorzugsweise mit einem Sender an eine Auswerteeinheit weitergeleitet werden.

Da eine schnelle Knochenbildung oder ein rasches Knochenwachstum erzielt werden soll, wird vorgeschlagen, durch Vibration z.B. der Antriebseinheit oder elektromagnetische Felder die Knochenbildung anzuregen. Eine Miniaturpumpe im Bereich des Distraktors kann dazu dienen, das Knochenwachstum anregende Stoffe wie beispielsweise Knochenhormone (z.B. BMP) oder Proteine (z.B. Prostaglandine) in den Knochenneubildungsbereich abzugeben.

Bevorzugte Ausführungsbeispiele der Erfindung sind in der Zeichnung dargestellt und werden im folgenden näher beschrieben.

Es zeigt,
- Figur 1: die Vorderansicht eines vertikal zweifach gewinkelten Distraktors,
- Figur 2: eine Seitenansicht des in Figur 1 gezeigten Distraktors,
- Figur 3: die Rückseite des in Figur 1 gezeigten Distraktors,
- Figur 4: die Rückseite des in Figur 1 gezeigten Distraktors in gestreckter Position,
- Figur 5: die Rückseite eines vertikal einfach gewinkelten Distraktors,
- Figur 6: die Vorderansicht eines horizontal zweifach gewinkelten Distraktors,
- Figur 7: eine Seitenansicht des in Figur 6 gezeigten Distraktors,
- Figur 8: die Rückansicht des in Figur 6 gezeigten Distraktors,
- Figur 9: die Draufsicht des in Figur 6 gezeigten Distraktors,
- Figur 10: eine Draufsicht des in Figur 6 gezeigten Distraktors in gestreckter Position,
- Figur 11: die Vorderansicht eines vertikal zweifach gewinkelten Distraktors,
- Figur 12: eine Seitenansicht des in Figur 11 gezeigten Distraktors,
- Figur 13: die Rückansicht des in Figur 11 gezeigten Distraktors,
- Figur 14: die Rückansicht des in Figur 11 gezeigten Distraktors in gestreckter Position,
- Figur 15: eine Rückansicht eines vertikal einfach gewinkelten Distraktors in gestreckter Position,
- Figur 16: die Vorderansicht eines horizontal zweifach und vertikal zweifach gewinkelten Distraktors,
- Figur 17: einen Schnitt längs der Linie 17 durch den in Figur 16 gezeigten Distraktor,
- Figur 18: eine Rückansicht des in Figur 16 gezeigten Distraktors,
- Figur 19: eine Vorderansicht des in Figur 16 gezeigten Distraktors in gestreckter Position,
- Figur 20: eine Draufsicht auf den in Figur 16 gezeigten Distraktor in gestreckter Position,
- Figur 21: eine Vorderansicht eines vertikal versetzt sowie horizontal einfach gewinkelten Distraktors in gestreckter Position,
- Figur 22: die Vorderansicht eines vertikal einfach gewinkelten Distraktors,
- Figur 23: eine Seitenansicht des in Figur 22 gezeigten Distraktors,
- Figur 24: eine Rückansicht des in Figur 22 gezeigten Distraktors,
- Figur 25: eine Vorderansicht des in Figur 22 gezeigten Distraktors in gestreckter Position,
- Figur 26: eine Seitenansicht eines einfach gewinkelten Distraktors,
- Figur 27: eine Vorderansicht des in Figur 26 gezeigten Distraktors,
- Figur 28: eine Rückansicht des in Figur 26 gezeigten Distraktors in gestreckter Position,
- Figur 29: eine Vorderansicht des in Figur 26 gezeigten Distraktors in gestreckter und vertikal einfach gewinkelter Position,
- Figur 30: eine Vorderansicht eines vertikal zweifach gewinkelten Distraktors,
- Figur 31: eine Seitenansicht des in Figur 30 gezeigten Distraktors,
- Figur 32: eine Rückansicht des in Figur 30 gezeigten Distraktors in gestreckter Position,
- Figur 33: eine Vorderansicht des in Figur 30 gezeigten Distraktors in gesteckter und zweifach gewinkelter Position,
- Figur 34: eine Seitenansicht eines vertikal einfach und horizontal zweifach abwinkelbaren Distraktors,
- Figur 35: eine Vorderansicht des in Figur 34 gezeigten Distraktors,
- Figur 36: eine Rückansicht des in Figur 34 gezeigten Distraktors in gestreckter Position,
- Figur 37: eine Rückansicht des in Figur 34 gezeigten Distraktors in vertikal einfach sowie horizontal einfach gewinkelter Position und
- Figur 38: eine Draufsicht auf den in Figur 34 gezeigten Distraktor in gestreckter und vertikal einfach sowie horizontal einfach gewinkelter Position.

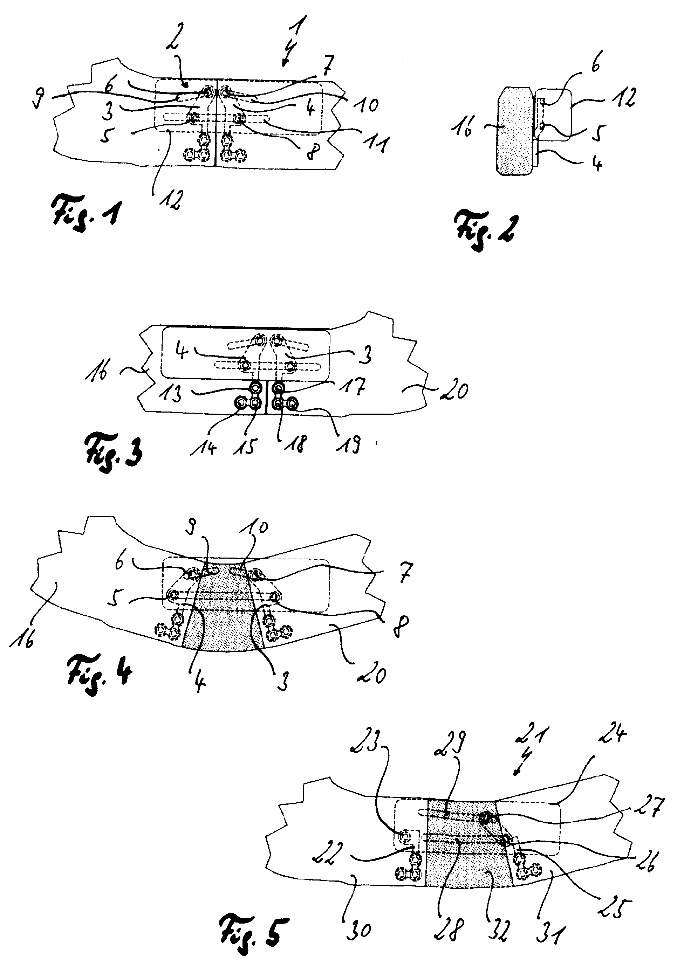

Der in Figur 1 gezeigte Knochendistraktor 1 hat eine Stelleinrichtung 2 mit zwei Knochenhalterungen 3 und 4. Die Knochenhalterungen 3 und 4 haben an ihrem oberen Ende jeweils zwei Anlenkpunkte 5, 6 bzw. 7, 8, die in Kurvenbahnen 9, 10, 11 geführt sind. Die in der Kurvenbahn 11 geführten Anlenkpunkte 5 und 8 bilden eine Verbindung zu einer im Gehäuse 12 angeordneten Antriebseinheit. Diese Antriebseinheit (nicht gezeigt) hat zwei Antriebsmaschinen, die derart auf die Anlenkpunkte 5 und 8 einwirken, daß diese Anlenkpunkte auf der geradlinig verlaufenden Kurvenbahn 11 auseinander bewegt werden können.

Bei einer Bewegung der Anlenkpunkte 5 und 8 in die, in Figur 4 gezeigte Position wandern die Anlenkpunkte 6 und 7 zwangsläufig auf den Kurvenbahnen 9 und 10 in entgegengesetzter Richtung, wodurch die zunächst parallel verlaufenden Knochenhalterungen 3 und 4 in die, in Figur 4 gezeigte, abgewinkelte Position verfahren werden.

Die Figur 2 zeigt die Knochenhalterung 4 mit ihren Anlenkpunkten 5 und 6, die die Verbindung zu der im Gehäuse 12 angeordneten Betriebseinheit bilden.

Die Figur 3 zeigt wie die unteren Enden der Knochenhalterungen 3 und 4 mittels im Dreick angeordneter Schrauben 13, 14 und 15 am Knochen 16 befestigt sind. Gegegnüberliegend ist die Knochenhalterung 3 mittels der Schrauben 17, 18 und 19 am Knochen 20 befestigt.

Bei einem Auseinanderbewegen der Knochenhalterungen 3 und 4 bewirken die Anlenkpunkte 5 bis 8 eine spezielle Bewegung der Knochenhalterungen, die durch die Kurvenbahnen 9, 10 und 11 vorbestimmt ist. Hierdurch werden die Knochen 16 und 20, an denen die Knochenhalterungen 3 und 4 befestigt sind, auf vorbestimmte genau definierte Art und Weise auseinander bewegt.

Als Antriebseinheit reicht im vorliegenden Fall eine einzige Antriebsmaschine aus, die beispielsweise über eine Spindel die Anlenkpunkte 5 und 8 auseinander bewegt. Es können jedoch auch zwei Antriebsmaschinen vewendet werden, die beispielsweise unabhängig von einander gesteuert die Knochenhalterungen bewegen können.

Die Figuren zeigen deutlich, daß die Knochenhalterungen 3 und 4 Hebel bilden, die jeweils an den zwei Punkten 5 und 6 bzw. 7 und 8 angelenkt sind. Hierdurch wird eine Bewegungskomponente der Bewegungsbahn der Knochenhalterungen durch die Kurvenbahnen 9 bzw. 10 bestimmt.

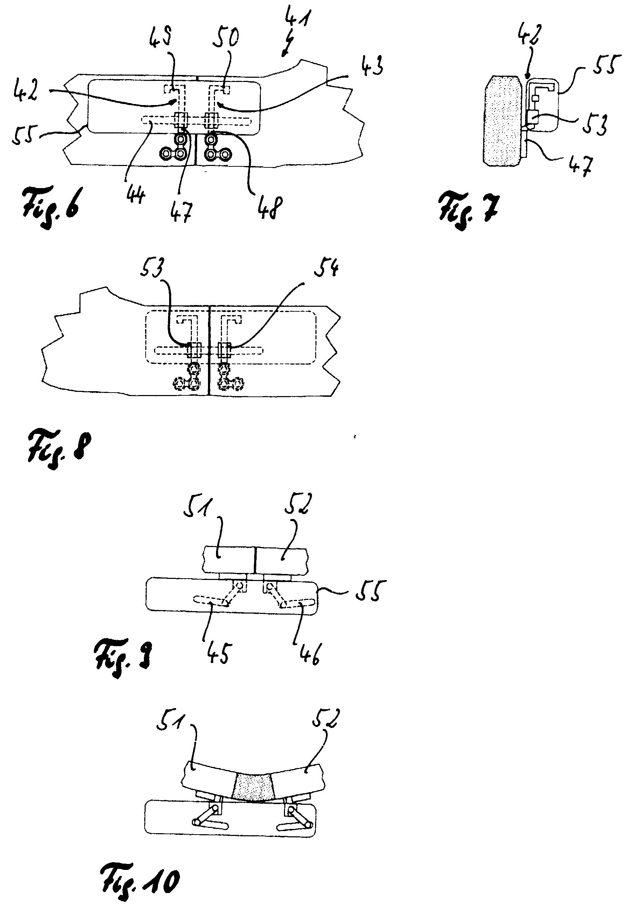

Die Figur 5 zeigt eine alternative Ausführungsform eines Knochendistraktors 21, bei dem eine Knochenhalterung 22 am Punkt 23 fest mit dem Gehäuse 24 verbunden ist, während die andere Knochenhalterung 25 mittels zwei Anlenkpunkten 26 und 27 in Kurvenbahnen 28 und 29 geführt ist.

Durch die Knochenhalterung 22 wird der Knochen 30 derart fest mit dem Gehäuse 24 verbunden, daß das Gehäuse 24 immer in einer fixierten Position relativ zum Knochenteil 30 angeordnet ist. Der Knochenteil 31 wird hingegen über die Knochenhalterung 25 entsprechend den Kurvenbahnen 28 und 29 in vorbestimmter Art in einem Winkel zum Knochenteil 30 bewegt. Das zwischen den Knochenteilen 30 und 31 angeordnete Knochenstück 32 zeigt den neue gebildeten Knochen, der während der Auseinanderbewegung der Knochenteile 30 und 31 vom Körper neu gebildet wird.

Der in den Figuren 1 bis 4 gezeigte Knochendistraktor ermöglicht ein Auseinanderbewegen der Knochenteile 16 und 20 und gleichzeitig eine zweifache vertikale Abwinklung, das heißt einen Winkel, der durch zwei relativ zum Gehäuse 12 bewegte Knochenhalterungen erzeugt wird. Der in Figur 5 gezeigte Knochendistraktor ermöglicht eine einfache vertikale Abwicklung, das heißt nur eine Knochenhalterung 25 wird relativ zum Gehäuse 24 bewegt.

In den Figuren 6 bis 10 ist ein weiterer Knochendistraktor 41 dargestellt, der eine zweifache Abwicklung in einer horizontalen Ebene, das heißt in einer zur zuvor beschriebenen vertikalen Ebene senkrecht liegende Ebene erlaubt.

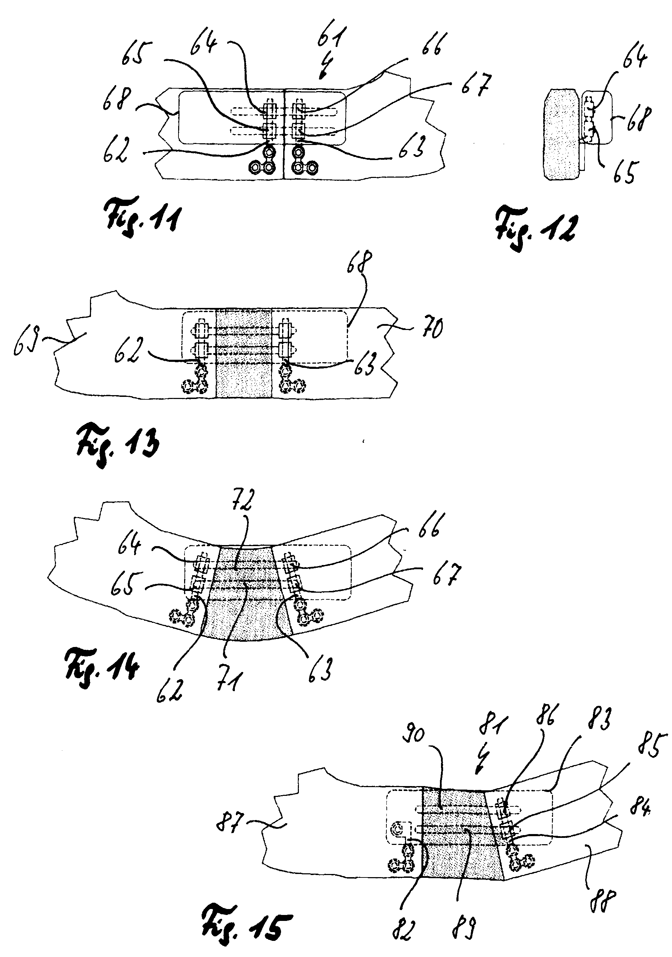

Hierzu sind die Knochenhalterungen 42 und 43 abgewinkelt ausgebildet, sodaß sie mit einer ersten geradlinigen Kurvenbahn 44 an der Vorderseite des Gehäuses 12 und mit jeweils einer Kurvenbahn 45 bzw. 46 an der in Figur 9 gezeigten Oberseite des Gehäuses 12 zusammenwirken. Die abgewinkelten Knochenhalterungen werden somit durch die im Gehäuse 12 angeordnete Antriebseinheit geradlinig links der Kurvenbahn 44 mit parallel zu einander angeordneten Schenkeln 47 und 48 auseinander bewegt und gleichzeitig bewirkt die Führung des abgewinkelt angeordneten Schenkels 49 bzw. 50 in der Kurvenbahn 45 bzw. 46 eine Drehung der Knochenhalterungen 42 bzw. 43 um die Längsachse der Schenkel 47 bzw. 48.

Die in Figur 10 gezeigte Darstellung des gestreckten Knochendistraktors zeigt, wie die Knochen einerseits auf ihrer ursprünglichen Verbindungsachse auseinandergezogen worden sind und andererseits durch die Einwirkung der Kurvenbahnen 45 und 46 abgewinkelt wurden, indem die Knochenhalterungen um die Längsachse ihrer Schenkel 47 bzw. 48 gedreht wurden.

Die Drehung der Schenkel 47 und 48 der Knochenhalterungen 42 und 43 wird durch eine verschiebbare Lagerung 53 bzw. 54 erreicht. Diese Lagerung erlaubt es, die fest mit den Knochenteilen 51 bzw. 52 verbundenen Knochenhalterugen 42 bzw. 43 um die Längsachse der Schenkel 47 bzw. 48 drehbar an Gehäuse 12 zu befestigen.

Eine andere Ausführungsform eines vertikal zweifach abwinkelbaren Knochendistraktors 61 ist in den Figuren 11 bis 14 dargestellt. Dieser Knochendistraktor 61 hat zwei Knochenhalterungen 62 und 63, die jeweils an zwei Anlenkpunkten 64, 65 bzw. 66, 67 mit einer im Gehäuse 68 angeordneten Antriebseinheit verbunden sind.

Die Figur 13 zeigt, wie diese Knochenhalterungen 62 und 63 über die im Gehäuse 68 angeordnete Antriebseinheit parallel verschoben werden können, um die an ihnen befestigten Knochenteile 69 und 70 auf einer geraden Achse zu verschieben.

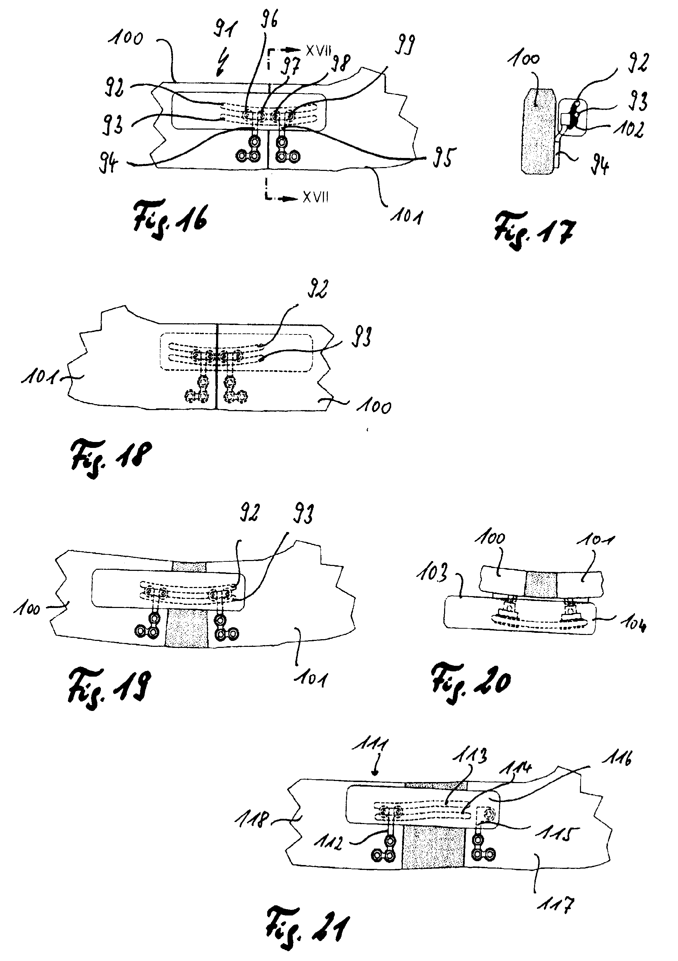

Die jeweils doppelte Anlenkung der Knochenhalterungen 62 und 63 ermöglicht jedoch auch ein abgewikeltes Auseinanderbewegen der Halterungen oder das Entstehen eines Winkels während des Auseinanderbewegens der Halterungen. Hierzu werden die in Figur 14 gezeigten unteren Anlenkpunkte 65 und 67 schneller auseinander bewegt als die darüberliegenden Anlenkpunkte 64 und 66. Diese unterschiedlichen Geschwindigkeiten können beispielsweise dadurch erzeugt werden, daß die Anlenkpunkte 64 und 66 auf einer Spindel mit einer geringeren Ganghöhe angeordnet sind und die darunterliegenden Anlenkpunkte 65 und 67 auf einer parallel verlaufenen Spidel mit einer höheren Ganghöhe. Bei gleichbleibender Drehgeschwindigkeit der Spindeln werden die Spindeln somit auf der unteren Führung 70 schneller auseinander bewegt als auf der oberen Führung 71, sodaß die Knochenhalterungen beim Auseinanderbewegen in eine abgewinkelte Position geführt werden. Die an den Knochenhalterungen befestigten Knochenteile 69 und 70 werden somit beim Auseinanderbewegen in eine abgewinkelte Position gebracht.

Der in der Figur 15 abgebildete Knochendistraktor 81 ist im wesentlichen wie der in den Figuren 11 bis 14 beschriebene Knochendistraktor aufbaut. Hierbei ist jedoch die rechte Knochenhalterung 82 fest mit dem Gehäuse 83 des Knochendistraktors 81 verbunden und nur die linke Knochenhalterung 84 ist zweimal an der Antriebseinheit (nicht gezeigt) an den Punkten 85 und 86 angelenkt. Dadurch entsteht ebenfalls eine geradlinige Bewegungskomponente beim Auseinanderbewegen der Knochehalterungen 82 und 84 und eine weitere Bewegungskomponente, die zur Abwinklung der Knochenstücke 87 und 88 relativ zueinander führt. Das Gehäuse 83 verbleibt jedoch in einer festgelegten Position relativ zum Knochenstück 87, da diese Teile über die Knochenhalterung 81 fest miteinander verbunden sind.

Die Figuren 11 bis 15 zeigen somit, daß auch mit zwei parallel zueinander angeordneten Kurvenbahnen 71 und 72 bzw. 89 und 90 eine geradlinige und eine zur ursprünglichen Position der Knochenteile 69 und 70 bzw. 87 und 88 abgewinkelte Bewegungskomponente erzielt werden kann.

Bei den beschriebenen Ausführungsformen hat die Antriebseinheit jeweils zwei Antriebsmaschinen, die unabhängig voneinander gesteuert werden können. Hierbei wirkt eine Antriebsmaschine auf die Anlenkpunkte 64 und 66 bzw. 86 und die andere Antriebsmaschine auf die Anlenkpunkte 65 und 67 bzw. 85.

Der in Figur 16 dargestellte Knochendistraktor 91 weist als Kurvenbahn an seiner Vorderseite zwei Schienen 92 und 93 auf, in denen die Knochenhalterungen 94 und 95 gehalten und geführt sind. Hierzu haben die Knochenhalterungen 94 und 95 an ihrem oberen Ende jeweils zwei Rollen 96, 97, 98, 99, die auf den Schienen 92 und 93 laufen. Am unteren Ende der Knochenhalterungen 94 und 95 ist jeweils ein Knochenteil 100, 101 befestigt, sodaß bei einem Auseinanderbewegen der Knochenhalterungen die Knochenteile 100 und 101 entsprechend der Führung der Schienen 92 und 93 auseinander bewegt werden.

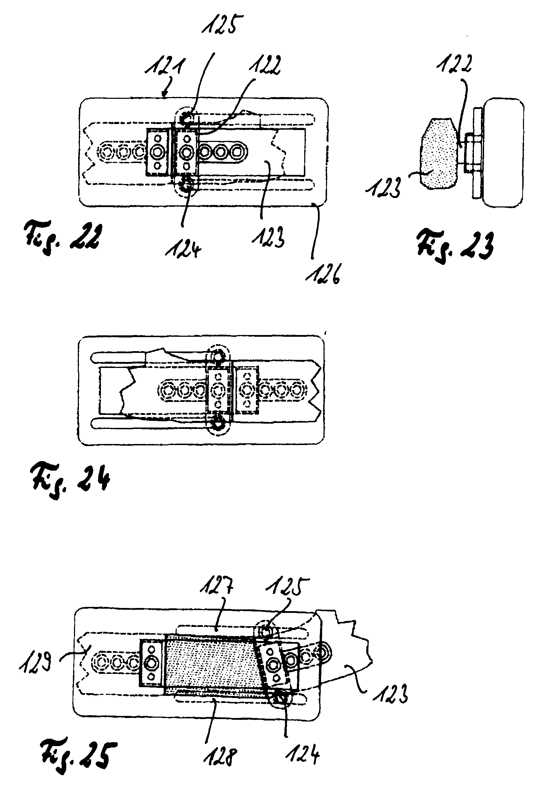

Die Figur 13 zeigt einen elastischen Überzug 102, der in den übrigen Figuren nicht gezeigt ist und eine Abdeckung der Schienen und der in den Schienen laufenden Rollen darstellt. Diese Abdeckung 102 soll verhindern, daß die Mechanik durch Schmutz oder Körperablagerungen beeinträchtigt wird, und sie soll den Patienten vor Verletzungen schützen. Auf den Schienen auseinandergefahrene Knochenhalterungen zeigt die Figur 19. Da die Schienen 92 und 93 leicht bogenförmig ausgebildet sind, werden die Knochen 100 und 102 sowohl mit einer geradlinigen als auch mit einer leicht nach oben weisenden Komponente auseinanderbewegt.

Die in Figur 20 gezeigte Draufsicht läßt erkennen, daß die Schienen 92 und 93 nicht parallel zur Vorderseite 103 des Gehäuses 104 verlaufen, sondern bogenförmig ausgebildet sind. Dies führt zu einer weiteren Bewegungskomponente, die beim Auseinanderbewegen der Knochenhalterungen auf die Knochen 100 und 101 einwirkt.

Während der in den Figuren 16 bis 20 gezeigte Knochendistraktor 91 bei seiner Bewegung sowohl horizontal als auch vertikal zweifach abgewinkelt werden kann, ist in Figur 21 ein Knochendistraktor 111 gezeigt, bei dem die eine Knochenhalterung 112 auf Schienen 113, 114 geführt ist und die andere Knochenhalterung 115 fest mit dem Gehäuse 116 verbunden ist. Das Gehäuse 116 und die darin angeordnete Antriebseinheit verbleiben somit in einer festgelegten Position relativ zum Knochenteil 117, während das andere Knochenteil 118 auf der durch die Schienen 113 und 114 vorgegebenen Bewegungsbahn geführt wird.

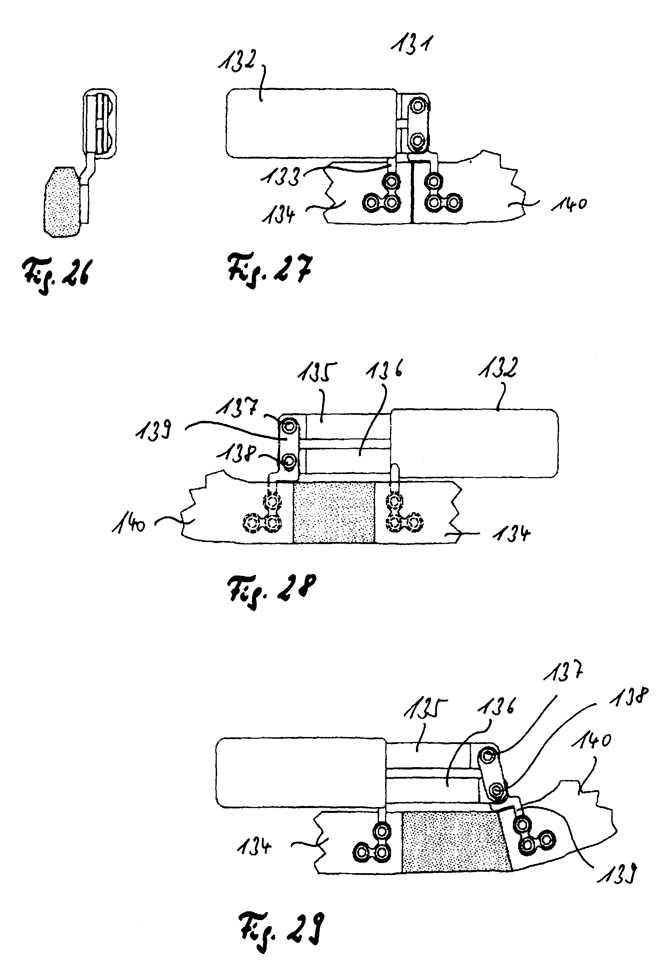

Ein in vertikaler Ausrichtung einfach abwinkelbarer Knochendistraktor 121 ist in den Figuren 22 bis 25 gezeigt. Die Knochenhalterung 122 ist hierbei fest mit dem Knochenstück 123 verbunden. Zwei Anlenkpunkte 124 und 125 verbinden die Knochenhalterung 122 mit der im Gehäuse 126 angeordneten Antriebseinheit. Diese Antriebseinheit (nicht gezeigt) ist so ausgebildet, daß sie mit unterschiedlicher Geschwindigkeit die Anlenkpunkte 124 und 125 auf geradlinigen parallel zueinanderliegenden Bahnen 127, 128 führt. Bei synchroner Bewegung der Anlenkpunkte 127 und 128 entsteht eine geradlinige Bewegung des Knochenteils 123 und sofern einer der Anlenkpunkte 124 schneller als sein gegenüberliegender Anlenkpunkt 125 bewegt wird, wirkt eine Bewegungskomponente auf das Knochenteil 123 ein, so daß es auf einer Bogenlinie vom anderen Knochenteil 129 wegbewegt wird.

Der in den Figuren 26 bis 29 gezeigte Knochendistraktor 131 besteht aus einem Gehäuse 132, das über eine erste Knochenhalterung 133 mit einem Kochenteil 134 starr verbunden ist. Die im Gehäuse angeordnete Antriebseinheit wirkt auf zwei Stößel 135, 136, die aus dem Gehäuse 132 herausfahrbar sind. Am Ende dieser Stößel ist über Anlenkpunkte 137, 138 eine weitere Knochenhalterung 139 befestigt. Diese Knochenhalterung ist fest mit dem anderen Stück 140 verbunden.

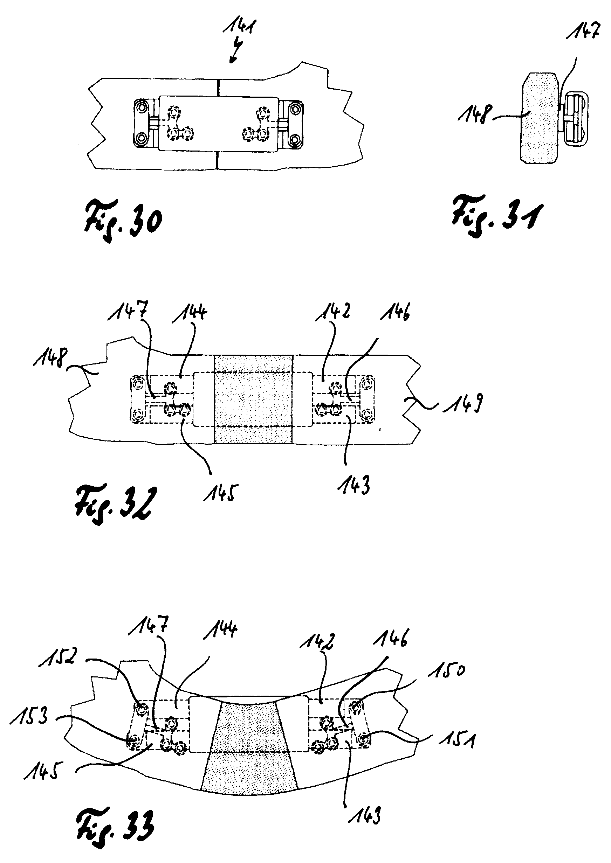

Bei einem Ausfahren der Stößel 135 und 136 aus dem Gehäuse 132 werden, wie in Figur 28 gezeigt, die Knochenteile 134 und 140 auf einer geradlinigen Bahn auseinanderbewegt. Die Figur 29 zeigt jedoch, daß die Stößel 135 und 136 nicht gleichförmig bewegt werden müssen und beispielsweise bei einer schnelleren Bewegung des Stößes 136 oder einer Bewegungspause am Stößel 135 eine weitere Bewegungskomponente auf das Knochenstück 140 einwirkt. Die Anlenkpunkte 137 und 138 führen somit zu einer Schrägstellung der Knochenhalterung 139 und somit seiner Abwinklung des Knochenstücks 140.

Eine zweifache Abwicklung in vertikaler Richtung wird durch den in den Figuren 30 bis 33 gezeigten Knochendistraktor 141 erreicht. Hierbei wirken gegenüberliegend jeweils zwei Stößel 142, 143 bzw. 144, 145 auf die Knochenhalterungen 146, 147. Diese Knochenhalterungen sind mit Knochenteilen 148, 149 verbunden, sodaß bei einem Ausfahren der Stößel die Knochenteile 148 und 149 auf einer geradlinigen Bewegungsbahn auseinander bewegt werden. Die Anlekpunkte 150, 151 bzw. 152, 153 an den Enden der Stößel 142, 143, 144, 145 ermöglichen eine unsynchrone Bewegung der Stößel und bewirken somit ein Abknicken der Knochenhalterungen 146, 147, wie es in Figur 33 gezeigt ist.

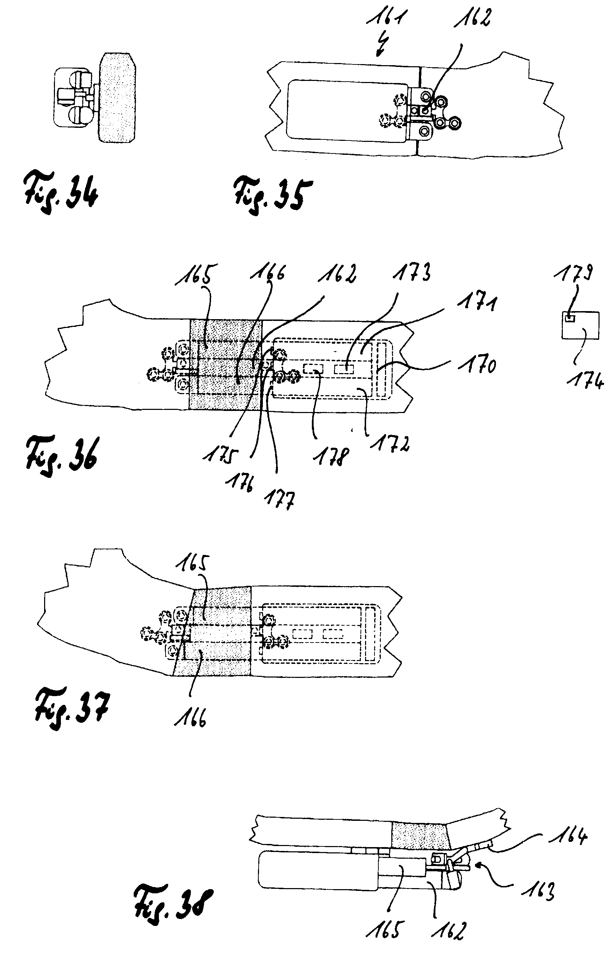

Die Figuren 34 bis 38 zeigen an einem weiteren Knochendistraktor 161, wie mit einem weiteren Stößel 162 eine zusätzliche Bewegungskomponente erreicht werden kann. Der Knochendistraktor 161 ist im wesentlichen wie der in den Figuren 27 bis 29 gezeigte Knochendistraktor 131 aufgebaut. Der zusätzliche Stößel 162 erlaubt somit neben der in Figur 37 gezeigten vertikalen einfachen Abwinklung eine in Figur 38 gezeigte horizontale einfache Anwinklung. Wie aus Figur 38 ersichtlich ist, wirkt der Stößel 162 auf eine Mechanik 163, die die Knochenhalterung 164 quer zur Bewegungsrichtung der Stößel 162, 165 und 166 bewegt.

In den vorliegenden Ausführungsbeispielen wurden als Antriebseinheit eine oder mehrere Elektro- oder Linearmotoren verwendet. Diese Motoren sind klein im Aufbau, lassen sich unabhängig voneinander steuern und ermöglichen eine hohe Leistungsabgabe.

Die Energieversorgung 170 ist in Figur 36 beispielhaft eingezeichnet und wird von einer wiederaufladbaren Batterie gebildet. Die Energieversorgung liegt somit in unmittelbarer Nähe neben den in Figur 36 beispielhaft eingezeichneten Antriebseinheiten 171 und 172.

Zur Steuerung der Antriebseinheit ist in Figur 36 ein Empfänger 173 gezeigt. Dieser Empfänger empfängt Signale eines Senders 174, den der Patient bei sich tragen kann oder der ausschließlich dem Arzt zur Verfügung steht.

Zur Feststellung der momentanen Position der Stößel 165, 166 und 162 ist jeweils ein Resolver oder ein Encoder 175, 176, 177 vorgesehen. Diese Meßeinrichtungen stehen mit einem Sender 178 in Verbindung, der die genauere Position an die Sende- und Auswerteeinheit 174 meldet. Hierzu ist in der Sende- und Auswerteeinheit 174 ein Empfänger 179 vorgesehen.

Die Ausführungsbeispiele zeigen im wesentlichen Knochenhalterungen, die in unterschiedlichen Richtungen lateral bewegt werden. Neben der bei allen Ausführungsbeispielen auftretenden geradlinigen Bewegungskomponente kann die weitere Bewegungskomponente entweder eine laterale oder eine rotatorische Bewegungskomponente sein. Es ist verständlich, daß eine Antriebseinheit auch eine rotatorische Bewegung auf eine Knochenhalterung ausüben kann, um beispielsweise bei einem gebogenen Unterkieferknochen neben der Vorwärtsbewegung auch eine Drehung des Knochens zu erreichen.

Mit Hilfe des multidirektionalen Distraktors kann neuer Knochen gebildet werden. Vorraussetzung dabei ist ein intaktes Periost (Knochenhaut) des zu verlängernden Knochens, der circulär vom Periost umschlossen sein muß. Durch die Verlängerung des Knochens in einer Richtung erfolgt der Knochenaufbau und Periostaufbau in diese Richtung, das Periost wächst mit. Diese Technik der osteogenen Knochendistraktion mit Gewinnung von neuem Knochen, kann man sich zur in-vivo Knochengewinnung als Knochenersatz für operative Knochen-Rekonstruktionen zunutze machen. Unter der Haut wird ein kleines gefässgestieltes Knochentransplantat (z.B. gefässgestieltes Fibula-Transplantat) samt Periost im Körper vorzugsweise am Schädel, Unterarm, Unterschenkel, etc. implantiert. Das gefässgestielte Knochentransplantat wird am Blutkreislauf im Transplantatbett angeschlossen und so aufbereitet, daß der Distraktor in das Transplantat integriert wird und ein in eine Richtung induziertes Wachstum des Transplantates bewirkt. Der Distraktor wird computergesteuert und durch die erwähnten Antriebseinheiten bewegt. Auf diese Weise kann man Knochen als Knochenersatzmittel für z.B. knöcherne Rekonstruktionen, z.B. nach einer Tumorresektion gewinnen. Dieser spezielle Distraktor ist mit einer medikamentösen Pumpe aus knochenbildenden Proteinen oder Knochenhormonen bestückt, der kontinuierliche Dosen der knochenanregenden Stoffe abgibt. Mittels der computergesteuerten Vorausberechnung kann mit Hilfe des multidirektionalen Distaktors eine gewünschte Form des neu gebildeten Knochens hergestellt werden. Auf diese Weise lassen sich Ersatzteilknochen für spätere Rekonstruktionen herstellen.

## Patentansprüche

1. Knochendistraktor (1) mit einer Stelleinrichtung (2), die mindestens zwei Knochenhalterungen (3, 4) aufweist, und einer Antriebseinheit mit mindestens einer Antriebsmaschine, ***dadurch gekennzeichnet, daß*** eine Knochenhalterung (3) relativ zu der anderen Knochenhalterungen (4) entlang einer Bewegungsbahn bewegbar ist, die zumindest eine geradlinige und eine weitere Bewegungskomponente umfaßt.

2. Knochendistraktor nach Anspruch 1, ***dadurch gekennzeichnet, daß*** zumindest eine Bewegungskomponente der Bewegungsbahn durch eine Kurvenbahn bewirkt wird.

3. Knochendistraktor nach Anspruch 1 oder 2, ***dadurch gekennzeichnet, daß*** die Knochenhalterungen (3, 4) Hebel aufweisen, die an mindestens zwei Punkten (5, 6, 7, 8) angelenkt sind.

4. Knochendistraktor nach einem der vorherigen Ansprüche, ***dadurch gekennzeichnet, daß*** die Knochenhalterungen (3, 4) Hebel aufweisen, die mit mindestens zwei Antriebsmaschinen in Wirkverbindung stehen.

5. Knochendistraktor nach einem der vorherigen Ansprüche, ***dadurch gekennzeichnet, daß*** die Antriebseinheit mindestens zwei parallel oder koaxial angeordnete Gestänge aufweist.

6. Knochendistraktor nach einem der vorherigen Ansprüche, ***dadurch gekennzeichnet, daß*** die Antriebseinheit einzeln steuerbare Antriebsmaschinen aufweist.

7. Knochendistraktor nach einem der vorherigen Ansprüche, ***dadurch gekennzeichnet, daß*** die Antriebseinheit mindestens einen Elektro-oder Liniarmotor (171, 172) aufweist.

8. Knochendistraktor nach einem der vorherigen, ***dadurch gekennzeichnet, daß*** die Energieversorgung (170) für die Antriebsmaschinen (171, 172) in der Nähe der Antriebseinheit angeordnet ist.

9. Knochendistraktor nach einem der vorherigen Ansprüche, ***dadurch gekennzeichnet, daß*** die Antriebseinheit einen Empfänger (173) aufweist.

10. Knochendistraktor nach einem der vorherigen Ansprüche, ***dadurch gekennzeichnet, daß*** die Stelleinrichtung (2) eine, vorzugsweise mit einem Sender versehene, Parametermeßeinrichtung (175, 176, 177) aufweist.
